(19) Europäisches Patentamt
European Patent Office
Office européen des brevets

(11) **EP 4 008 368 A1**

(12) **EUROPEAN PATENT APPLICATION**
published in accordance with Art. 153(4) EPC

(43) Date of publication:
**08.06.2022 Bulletin 2022/23**

(51) International Patent Classification (IPC):
*A61L 27/22* (2006.01)    *A61K 35/12* (2015.01)
*A61K 35/22* (2015.01)    *A61K 35/30* (2015.01)
*A61K 35/34* (2015.01)    *A61K 35/36* (2015.01)
*A61K 35/38* (2015.01)    *A61K 35/50* (2015.01)
*A61K 38/36* (2006.01)    *A61L 27/36* (2006.01)
*A61L 27/58* (2006.01)    *A61P 9/00* (2006.01)
*A61P 43/00* (2006.01)

(21) Application number: 20847207.6

(22) Date of filing: 31.07.2020

(52) Cooperative Patent Classification (CPC):
**A61K 35/12; A61K 35/22; A61K 35/30;**
**A61K 35/34; A61K 35/36; A61K 35/38;**
**A61K 35/50; A61K 38/36; A61L 27/22;**
**A61L 27/36; A61L 27/58; A61P 9/00; A61P 43/00**

(86) International application number:
**PCT/JP2020/029532**

(87) International publication number:
**WO 2021/020576 (04.02.2021 Gazette 2021/05)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **01.08.2019 JP 2019142482**

(71) Applicant: **KM Biologics Co., Ltd.**
**Kumamoto-shi, Kumamoto 860-8568 (JP)**

(72) Inventors:
• **SAGA, Hideki**
**Kikuchi-shi, Kumamoto 869-1298 (JP)**
• **INUI, Minako**
**Kikuchi-shi, Kumamoto 869-1298 (JP)**
• **OGURA, Noriko**
**Kikuchi-shi, Kumamoto 869-1298 (JP)**

(74) Representative: **Vossius & Partner**
**Patentanwälte Rechtsanwälte mbB**
**Siebertstraße 3**
**81675 München (DE)**

(54) **TISSUE FIBROSIS INHIBITOR IN WHICH BIOCOMPATIBLE POLYMER IS USED**

(57) A problem to be solved by the present invention is to provide a fibrosis inhibitor that solves the problem of inhibiting fibrosis of an organ or tissue surface, and especially of inhibiting fibrosis of an epicardial surface. Furthermore, by inhibiting fibrosis, the present invention prevents or reduces subsequent development of adhesions to avoid organ or tissue damage during re-operation. Provided is a fibrosis inhibitor for inhibiting fibrosis of a tissue by fixing a biocompatible polymer to a tissue where it is desirable to inhibit fibrosis.

[Fig.6]

**Description**

TECHNICAL FIELD

The present invention relates to a fibrosis inhibitor for a tissue after a surgical operation. More specifically, the present invention relates to a fibrosis inhibitor characterized by using a biocompatible polymer, which can also be used for the inhibition of the occurrence of adhesion of tissues.

BACKGROUND ART

Fibrosis is a phenomenon that connective tissues are accumulated in a tissue, and is caused as the result of the excessive production and accumulation of connective tissues such as an extracellular matrix mainly composed of collagen in a tissue. Fibrosis is observed in heart, lung, liver, pancreas, kidney and the like. Among these, fibrosis in heart causes the hardening of cardiac muscles and impairs the functions of the heart, although the fibrosis can prevent cardiac rupture in the heart after the death of cardiac muscle cell. When the cardiac malfunction becomes persistent, the enlargement of the heart occurs so as to compensate the cardiac malfunction. Therefore, it is quite important to control fibrosis. When physical stress such as an autologous tissue and an artificial object or a chemical reaction is applied to the surface of an organ such as epicardium (the surface of the heart) or a tissue, the formation of fibers is increased on the surface of the tissue, leading to the enlargement of the tissue. In a reoperative surgery, particularly in a reoperative cardiac surgery, the visibility of epicardium is remarkably deteriorated due to fibrosis, the running of a coronary artery becomes unclear, and therefore there is a risk of leading to the damage of an organ such as heart and a blood vessel or a tissue. There has not been found yet any fibrosis inhibitor which can meet the needs of surgeons. Therefore, the production of a fibrosis inhibitor which can inhibit the fibrosis of the surface of an organ or a tissue, particularly the surface of epicardium, has been demanded.

Besides fibrosis, another risk which may cause a damage in an organ or a tissue is the adhesion of a tissue after surgery. The adhesion of a tissue after surgery occurs in various fields, including cardiology as well as digestive system surgery, orthopedic surgery, gynecology and obstetrics, ophthalmology and the like. For example, the adhesion in abdominal cavity after an abdominal surgery is a physiological biological repairing reaction, and it is difficult to absolutely prevent the occurrence of the adhesion. The adhesion occurring after surgery causes adhesive intestinal obstruction with some frequency. It is believed that this frequency is proportional to some extent to the duration of surgery and the amount of bleeding. In order to prevent or reduce the occurrence of adhesion, it has been required to minimize detachment/bleeding and operative duration, to prevent contamination during operation, and to minimize remaining foreign matters by careful surgical operation by a surgeon, to use an absorbable suture thread, to perform proper drainage, to prevent infection, to perform early postoperative ambulation, and so on. In an emergency contaminated operation, it has been also required to perform sufficient intraperitoneal irrigation and the change in a body position and to use an antibiotic after operation. However, although these countermeasures are effective to some extent on the prevention of adhesive intestinal obstruction, it is impossible to prevent the occurrence of intestinal obstruction completely by these countermeasures (Non-Patent Document 1).

According to the recent studies, it is suggested that: mesothelial cells, which are film-like tissues that cover the surface of a body cavity such as chest cavity, pericardium and abdominal cavity, produce interleukin-6 at an operative stress site; neutrophils produce tumor necrosis factor TNF-α and transforming growth factor TGF-β by the action of interleukin-6; and the mesothelial cells are replaced by fibrosis by the action of this TGF-β to serve as a main body of the formation of adhesion (Non-Patent Document 2) .

In the field of cardiology, the adhesion of epicardium to chest wall or the adhesion of pericardium to epicardium after surgery increases the risk of damage to the heart or a large blood vessel during reoperative surgery (Non-Patent Document 3).

In a cardiac surgery, an autologous pericardium is used. However, when closing cannot be achieved using only the autologous pericardium, various types of pericardial substitutes are used. Examples of the pericardial substitute include bovine pericardium, a silicone coated polyester fabric, and a polytetrafluoroethylene (PTFE) sheet (Non-Patent Document 4).

In a pericardium defect site in human after an open-heart surgery, for the purpose of avoiding the occurrence of damage to the heart and a large blood vessel during cardiac tamponade in a postoperative acute phase and sternotomy in a reoperative surgery in a late postoperative phase, a PTFE sheet having a thickness of 0.1mm has been used frequently as a pericardial substitute. The results of the reoperative surgery in the long-term late postoperative phase are quite different from those in a case where the heart was covered with an autologous pericardium. The non-absorbable PTFE sheet, which has a structure into which a biological tissue cannot invade, can be removed easily during a reoperative surgery. However, both of the heart side and the sternum side of the PTFE sheet are covered with fiber tissues to such a level that the anatomical structure of each of the fiber tissues cannot be confirmed. Particularly in a case where a burden by a residual lesion is found, the thickening of the fiber tissues appears remarkably. When a broad area of the surface of the heart is supplemented with a sheet during an initial surgery, it is no wonder that any diastolic failure is observed even if the diagnosis of constrictive pericarditis (CP) is not given (Non-Patent Document 5). Furthermore, experimental data for the case are also publicly known (Non-Patent Documents 6 to 8).

When a PTFE sheet is transplanted as a pericardial substitute, an evidence of an inflammation induced by a foreign-body reaction is strongly observed, the inflammatory reaction becomes remarkable 4 weeks after the transplantation and is weakened 12 weeks after the transplantation. As the result of the transplantation, a very thick fibrous connective tissue film is formed on the epicardium side of the PTFE sheet. This PTFE sheet is useful, because the sheet covers the heart and a large blood vessel so as not to damage the heart and the large blood vessel during sternotomy that is performed when a reoperative surgery becomes necessary. However, in the PTFF sheet, the problem is the influence of the foreign-body reaction in the PTFE sheet when a reoperative surgery is not necessary, particularly diastolic failure including CP. When the PTFE sheet is removed, a region bounded by the sheet can be discriminated, and therefore the PTFE sheet is often believed to rarely cause inflammation with surrounding tissues. However, as a matter of fact, a PTFE sheet is likely to cause an inflammatory reaction (Non-Patent Document 5). Furthermore, it is also reported that the fibrosis of epicardium which is caused by a PTFE sheet impairs the visibility of the heart (Non-Patent Document 9).

The fibrosis of epicardium is induced not only by a PTFE sheet but also by a pericardium defect case where an autologous pericardium is resected or an autologous pericardium closure case. In an experiment using a rabbit model, there has been reported about the occurrence of fibrosis in an expanded polytetrafluoroethylene (ePTFE) closure case and a pericardium defect case where an autologous pericardium is resected (Non-Patent Document 10) . In an experiment using a dog model, there has been reported about the occurrence of fibrosis in an ePTFE closure case and an autologous pericardium closure case (Non-Patent Document 11).

Meanwhile, a case where a fibrin glue that is a biological tissue adhesive agent is used for adhesion inhibition purposes has also been reported (Non-Patent Documents 12 and 13). However, a fibrin glue cannot inhibit the adhesion of tissues completely (Patent Documents 1 to 3), and therefore serious problems which surgeons are worried about have not been solved yet.

A decellularized biological tissue and a decellularized organ (which is collectively referred to as a "decellularized tissue", hereinafter) is a matrix produced by removing cell components from a biological tissue or an organ in a human body or an animal of a different species, and has been focused as a cell scaffold material or a wound healing promoting material which can be used for transplantation or regenerative medicine. A component other than cells in a biological tissue, i.e., an extracellular matrix (ECM), is conserved with respect to the structure and composition thereof among organism species, and it is found clearly that the component does not induce immune rejection among almost all of organism species. Furthermore, it is also widely known that an ECM is involved in the differentiation of a cell, and it is reported that a cell cultured on an ECM in a specific tissue is differentiated into a cell of the tissue. From these findings, a decellularized tissue constituting an ECM does not undergo immune rejection after transplantation, and is expected to be regenerated by cells of a recipient (Non-Patent Document 14) .

PRIOR ART DOCUMENTS

PATENT DOCUMENTS

Patent Document 1: JP-T-11-502431
Patent Document 2: JP-A-2001-327592
Patent Document 3: JP-T-2003-500170
Patent Document 4: JP-4092397
Patent Document 5: JP-A1-2008-111530
Patent Document 6: JP-A-2009-50297

NON-PATENT DOCUMENTS

Non-Patent Document 1: Tsuneo Fukushima, et al., Surgical Therapy, 2006; 94(6):919-924
Non-Patent Document 2: Uyama N. et al., Sci Rep. 2019; 26;9(1) :17558
Non-Patent Document 3: Dobell AR., et al., Ann Thorac Surg.1984; 37:273-8
Non-Patent Document 4: Ozeren M. et al., Cardiovasc. Surg. 2002; 10(5):489-493

Non-Patent Document 5: Tetsuya Kitagawa, PEDIATRIC CARDIOLOGY and CARDIAC SURGERY. 2004; 20(6):632-633
Non-Patent Document 6: Liermann A. et al., Helv Chir Acta. 1992; 58:515-519
Non-Patent Document 7: Muralidharan S. et al., J Biomed Mater Res. 1991; 25:1201-1209
Non-Patent Document 8: Bunton RW. et al., J Thorac Cardiovasc Surg. 1990; 100:99-107
Non-Patent Document 9: Jukka T. et al., Interactive cardiovascular and thoracic surgery. 2011; 12(2):270-272
Non-Patent Document 10: Kaushal S. et al., Thorac Cardiovasc Surg. 2011; 141:789-795
Non-Patent Document 11: Naito Y. et al., J Thorac Cardiovasc Surg. 2008; 135:850-856
Non-Patent Document 12: Takamichi Sato et al., Obstetrics and Gynecology, 1990; 57(12):2398-2404
Non-Patent Document 13: Kiyoshi Okuda et al., The World of Obstetrics and Gynecology, 1993; 45(9):759-764
Non-Patent Document 14: Akio Kishida, Organ Biology, 2018; 25 (1) : 27-34
Non-Patent Document 15: Singelyn J.M., et al., Biomaterials, 2009, 30, 5409-5416
Non-Patent Document 16: Singelyn J.M,, et al., J. Am. Coll. Cardiol., 2012, 59, 751-763
Non-Patent Document 17: Sonya B., et al., Sci. Transl. Med., 2013, 5, 173ra25
Non-Patent Document 18: Sasaki S., et al., Mol. Vis., 2009, 15, 2022-2028
Non-Patent Document 19: Yoshihide H., et al., Biomaterials, 2010, 31, 3941-3949
Non-Patent Document 20: Seiichi F., et al., Biomaterials, 2010, 31, 3590-3595
Non-Patent Document 21: Negishi J., et al., J. Artif. Organs, 2011, 14, 223-231
Non-Patent Document 22: Hirashima M., et al., J. Biochem., 2016, 159, 261-270
Non-Patent Document 23: Meta A., et al., J. Biosci. Bioeng, 2015, 120, 432-437

## SUMMARY OF THE INVENTION

### PROBLEMS TO BE SOLVED BY THE INVENTION

Problems to be solved by the present invention is (1) the inhibition of the fibrosis of the surface of an organ or a tissue, particularly (2) the inhibition of the fibrosis of the surface of epicardium, and is to provide a fibrosis inhibitor which can solve the problems. Another problem is to prevent and reduce the occurrence of the subsequent adhesion by inhibiting the fibrosis to avoid the damage of the organ or the tissue during a reoperative surgery.

### SOLUTIONS TO THE PROBLEMS

The present inventors have made extensive and intensive studies. As a result, the present inventors have found a fibrosis inhibitor by which a biocompatible polymer is fixed to a tissue fibrosis of which is to be inhibited, thereby the fibrosis of the tissue is inhibited, and they have found that the fibrosis inhibitor can solve the problems.

The present invention includes the following aspects:

(1) A tissue fibrosis inhibitor comprising a biocompatible polymer.
(2) The fibrosis inhibitor according to (1), wherein the biocompatible polymer is fibrin or a dextrin gel.
(3) A tissue fibrosis inhibition kit comprising a combination of a biocompatible polymer and a cell scaffold material.
(4) The fibrosis inhibition kit according to (3), wherein the biocompatible polymer is fixed to one tissue fibrosis of which is to be inhibited, and prosthesis is made with the cell scaffold material for a tissue defect site in the other tissue.
(5) The fibrosis inhibition kit according to (3) or (4), wherein the biocompatible polymer is fibrin or a dextrin gel.
(6) The fibrosis inhibition kit according to any one of (3) to (5), wherein the cell scaffold material is a decellularized tissue.
(7) The fibrosis inhibition kit according to (6), wherein the decellularized tissue is a tissue produced by decellularizing a biological tissue selected from the group consisting of a small intestinal submucosa, pericardium, bladder, amnion, dura mater, peritoneum, greater omentum, thoracic diaphragm, fascia, dermis and skin.
(8) The fibrosis inhibition kit according to any one of (3) to (7), which promotes the self-organization of the cell scaffold material.
(9) The fibrosis inhibition kit according to any one of (3) to (8), wherein the tissue fibrosis of which is to be inhibited is epicardium.
(10) A tissue fibrosis inhibition kit comprising a combination of a biocompatible polymer and a pericardial substitute.
(11) The fibrosis inhibition kit according to (10), wherein the biocompatible polymer is fixed to one tissue fibrosis of which is to be inhibited, and prosthesis is made with the pericardial substitute for a tissue defect site in the other tissue.
(12) The fibrosis inhibition kit according to (10) or (11), wherein the biocompatible polymer is fibrin or a dextrin gel.
(13) The fibrosis inhibition kit according to any one of (10) to (12), wherein the pericardial substitute is a decellularized tissue.

(14) The fibrosis inhibition kit according to (13), wherein the decellularized tissue is a tissue produced by decellularizing a biological tissue selected from the group consisting of a small intestinal submucosa, pericardium, bladder, amnion, dura mater, peritoneum, greater omentum, thoracic diaphragm, fascia, dermis and skin.

(15) The fibrosis inhibition kit according to any one of (10) to (14), which promotes the self-organization of the pericardial substitute.

(16) The fibrosis inhibition kit according to any one of (10) to (15), wherein the tissue fibrosis of which is to be inhibited is pericardium.

(17) An adhesion barrier kit, wherein the biocompatible polymer is fixed to one tissue fibrosis of which is to be inhibited, and prosthesis is made with the cell scaffold material or the pericardial substitute for a tissue defect site in the other tissue.

(18) The adhesion barrier kit according to (17), wherein the biocompatible polymer is fibrin or a dextrin gel.

(19) The adhesion barrier kit according to (17), wherein the cell scaffold material or the pericardial substitute is a decellularized tissue.

(20) The adhesion barrier kit according to (19), wherein the decellularized tissue is a tissue produced by decellularizing a biological tissue selected from the group consisting of a small intestinal submucosa, pericardium, bladder, amnion, dura mater, peritoneum, greater omentum, thoracic diaphragm, fascia, dermis and skin.

(21) The adhesion barrier kit according to any one of (17) to (20), which promotes the self-organization of the cell scaffold material or the pericardial substitute.

(22) The adhesion barrier kit according to any one of (17) to (21), wherein the tissue to be inhibited from adhesion is epicardium.

EFFECTS OF THE INVENTION

The fibrosis inhibitor according to the present invention has an effect to inhibit the fibrosis of the surface of an organ or a tissue. The fibrosis inhibitor of the present invention inhibits the fibrosis of the surface of an organ or a tissue, particularly the surface of epicardium, to improve the visibility of the epicardium, and thereby can reduce the damages of the heart or a blood vessel during a reoperative surgery. Furthermore, the fibrosis inhibitor of the present invention can inhibit an inflammatory reaction that induces fibrosis. Therefore, the fibrosis inhibitor is also expected to have an effect to reduce adhesion associated with an inflammatory reaction and may be used as an adhesion inhibitor.

BRIEF DESCRIPTION OF THE DRAWINGS

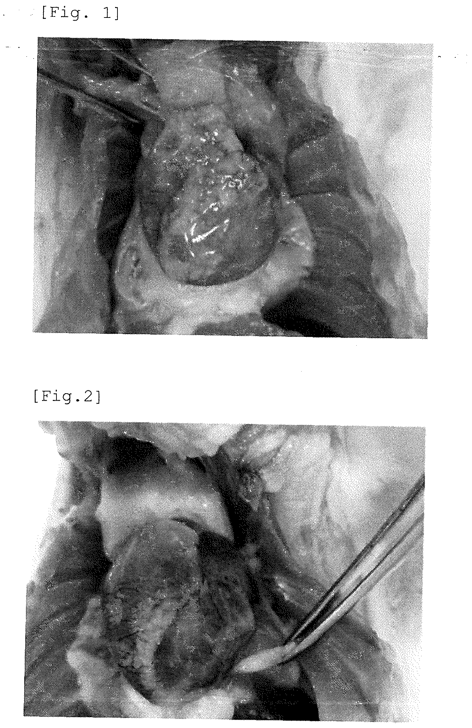

Fig. 1 is a photograph showing the evaluation of a rabbit cardiac fibrosis model (a fibrosis inhibitor group) after the observation for three months.

Fig. 2 is a photograph showing the evaluation of a rabbit cardiac fibrosis model (a fibrosis inhibition kit group) after the observation for three months.

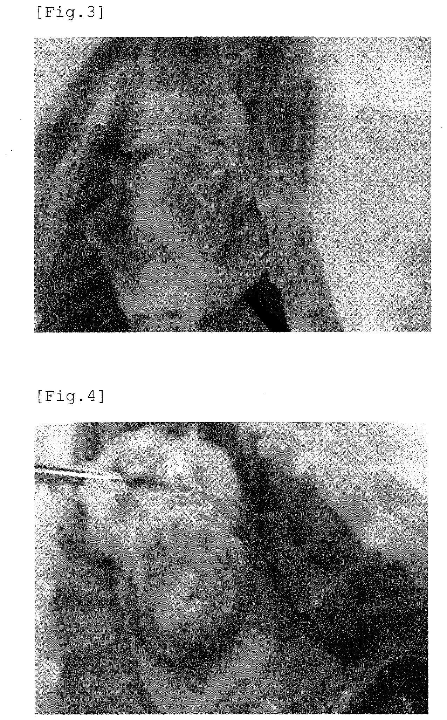

Fig. 3 is a photograph showing the evaluation of a rabbit cardiac fibrosis model (a control group/pericardium defect) after the observation for three months.

Fig. 4 is a photograph showing the evaluation of a rabbit cardiac fibrosis model (a control group/autologous pericardium closure) after the observation for three months.

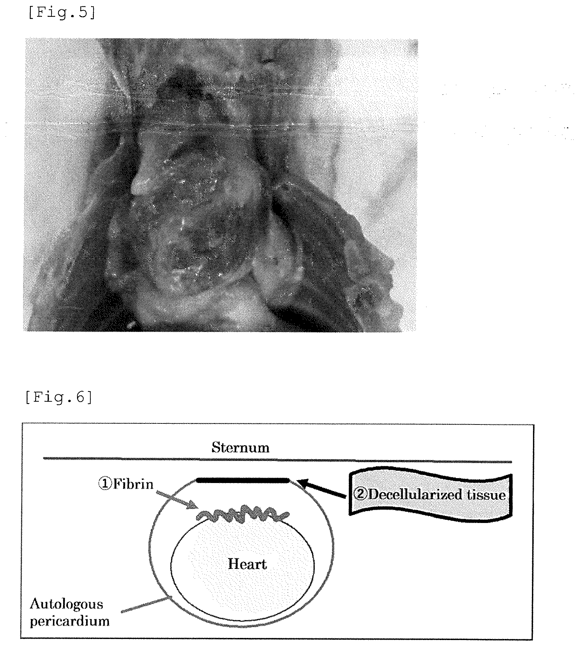

Fig. 5 is a photograph showing the evaluation of a rabbit cardiac fibrosis model (a decellularized tissue alone group) after the observation for three months.

Fig. 6 shows an example of the method for using a fibrosis inhibition kit and an adhesion barrier kit (a case where (1) fibrin is applied and fixed to epicardium in the heart and then (2) a decellularized tissue is sutured and fixed to a pericardium defect site located between epicardium and a sternum).

Fig. 7 is a photograph showing the pathology of a rabbit cardiac model (a fibrosis inhibition kit and adhesion barrier kit group) after the observation for three months.

Fig. 8 is a photograph showing the pathology of a rabbit cardiac model (a decellularized tissue alone group) after the observation for three months.

Fig. 9 is a photograph showing the pathology of a rabbit cardiac model (a fibrin-(decellularized tissue) complex group) after the observation for three months.

EMBODIMENTS OF THE INVENTION

The present invention includes: a biocompatible polymer that can be used as a fibrosis inhibitor; and a cell scaffold material.

The term "biocompatible polymer" as used herein refers to a polymer which exerts less immune reactions in vivo. Examples of the biocompatible polymer include, but are not limited to, alginic acid, chitin, chitosan, glycosaminoglycan, collagen, chondroitin sulfate, cellulose, gelatin, dextran, dextrin, a glycoprotein, hyaluronic acid, fibrinogen, fibrin, fibronectin, heparin, and heparan. As the biocompatible polymer, fibrin or a dextrin gel is particularly preferable.

The term "cell scaffold material" as used herein refers to a material which promotes the adhesion/proliferation of cells.

The decellularized tissue can be obtained by collecting a biological tissue from an animal (donor) and then subjecting the biological tissue to a decellularization treatment. By subjecting the biological tissue to a decellularization treatment, cells or pathogens (viruses, bacteria) from the donor can be removed from the biological tissue. As a result, even when the biological tissue is transplanted into an animal that is of a different species from that of the donor, the occurrence of a heterologous immune reaction can be inhibited. For these reasons, the type of the animal from which the biological tissue is to be collected is not particularly limited. Meanwhile, it is preferred to obtain the biological tissue easily. Therefore, the animal is preferably a non-human animal, particularly preferably a mammalian livestock or an avian livestock. Examples of the mammalian livestock include cattle, horse, camel, llama, ass, yak, sheep, pig, goat, deer, alpaca, dog, raccoon dog, weasel, fox, cat, rabbit, hamster, guinea pig, rat, squirrel and raccoon. Examples of the avian livestock include parakeet, parrot, chicken, duck, turkey, goose, guinea fowl, pheasant, ostrich and quail. Among these animals, pig is preferred from the viewpoint of availability.

An example of the biological tissue is a tissue which has an extracellular matrix structure and can be subjected to a decellularization treatment. An example of the tissue is a tissue selected from the group consisting of a small intestinal submucosa, bladder, amnion, dura mater, peritoneum, thoracic diaphragm, fascia, dermis, skin, liver, kidney, urinary duct, urethra, tongue, tonsilla, esophagus, stomach, greater omentum, small intestine, large intestine, anus, pancreas, heart, pericardium, blood vessel, spleen, lung, brain, bone, spinal cord, cartilage, testis, uterus, uterine tube, ovary, placenta, cornea, skeletal muscle, a tendon, a nerve and the like. When the decellularized tissue has a sheet-like form, the decellularized tissue can be applied into a living body easily. Therefore, one preferred example of the tissue is a tissue selected from the group consisting of a small intestinal submucosa, bladder, amnion, dura mater, peritoneum, greater omentum, thoracic diaphragm, fascia, dermis and skin. A decellularized tissue that does not have a sheet-like form is not unacceptable, because the decellularized tissue just has to be processed into a sheet-like form.

As the method for collecting the biological tissue from the animal, a method suitable for the animal species of the donor or the biological tissue may be employed. The biological tissue collected from the animal is subjected to a decellularization treatment. The decellularization treatment is not particularly limited, as long as cells or pathogens originated from the donor can be removed. Examples of the method include a treatment with a surfactant (Non-Patent Documents 15 to 21, Patent Documents 4 to 6), and the method may be selected appropriately depending on the animal spices of the donor and the type of the biological tissue.

When the biocompatible polymer is combined with the decellularized tissue, the fixation of the biocompatible polymer to a tissue fibrosis of which is to be inhibited can be achieved by the gelation of the biocompatible polymer by itself. Examples of the method for fixing fibrin, which is one of biocompatible polymers, include: a method in which fibrin is fixed to a tissue fibrosis of which is to be inhibited by applying fibrin to the tissue; and a method in which fibrin is fixed to the tissue by spraying a powder that is a fibrin constituent component (a fibrinogen powder, a thrombin powder) to the tissue. For example, when pericardium is defected during a cardiac surgery, fibrosis of epicardium and the like become a problem. For the purpose of inhibiting the fibrosis of epicardium, it is exemplified that fibrin is fixed to the epicardium while the decellularized tissue is sutured and fixed to an autologous pericardium that remains for the purpose of reducing the contact with a sternum. Fibrin is fixed to a tissue fibrosis of which is to be inhibited, more specifically epicardium, while prosthesis is made with the decellularized tissue for a pericardium defect site located between the epicardium and a sternum. In this manner, the effect as a fibrosis inhibition kit can be exerted. In the fixation of fibrin, it may be possible to apply either one of fibrinogen or thrombin firstly and to apply the other secondly, or it may also be possible to apply both of them simultaneously. A spraying application using a spraying device may also be selected. The fixation of fibrin is completed when the slight clouding of a transparent gel is observed. An example of the tissue fibrosis of which is to be inhibited is epicardium, as mentioned above. However, examples of the tissue are not limited to epicardium. In addition to epicardium, it is believed that the occurrence of fibrosis can be inhibited in a tissue of the lung or the like.

The concentration of fibrinogen or thrombin at the time of application of fibrin is not particularly limited, as long as fibrin can be formed. For example, the concentration of fibrinogen is 4 mg/mL to 160 mg/mL, preferably 10 mg/mL to 80 mg/mL. For example, the concentration of thrombin is 1 U/mL to 1200 U/mL, preferably 60 U/mL to 600 U/mL.

Fibrin is a glue-like coagulate formed as the result of the interaction of fibrinogen with thrombin that is an enzyme, and is a drug product that can be utilized for the closing of a tissue, the adhesion of a damaged site in an organ, the arrest of bleeding and the like. The type of fibrin is not particularly limited, and may be one in which constituent components such as fibrinogen and thrombin are derived from blood or may be prepared by a recombination technique. A preferred example is BOLHEAL for tissue adhesion (registered trade mark, KM Biologics Co., Ltd.).

The present invention also includes a fibrosis inhibition kit in which fibrin and the decellularized tissue are prepared separately. The kit is configured such that a physician applies fibrin and the decellularized tissue upon use. When the decellularized tissue is lyophilized, the decellularized tissue is impregnated with a solvent to restore the decellularized tissue and the restored decellularized tissue is applied to an affected site. Therefore, the kit may include a solvent for restoring use.

The animal to be used for the production of a cardiac fibrosis model is not particularly limited, and is preferably a rabbit, more preferably a 5- to 6-month-old Japanese white rabbit. In the production of the model, the thorax of the animal is opened by an incision under anesthesia and then a metallic raspatory is brought into contact with the heart or the epicardium. The duration of contact with the metallic raspatory is not particularly limited, and is, for example, 10 to 20 minutes. After the treatment with the metallic raspatory, the heart may be dried with a drier (by supplying air).

An example of the method for evaluating the inhibition of fibrosis is rating by visual observation. For example, in the case of the evaluation of the inhibition of fibrosis in epicardium, the evaluation may be carried out by the rating at fibrosis grade 0 (fibrosis is not observed), fibrosis grade 1 (the pericardium can be visually observed) and fibrosis grade 2 (the epicardium cannot be visually observed). In this evaluation method, the determination may be performed depending on whether or not blood vessels on the epicardium can be visually observed. When the epicardium is whitened and blood vessels cannot be observed, it may be determined that fibrosis is not inhibited satisfactorily. In addition, a method in which a region with fibrosis grade 2 is compared with a control group to evaluate the fibrosis inhibition effect can also be employed preferably.

The wording "self-organization of a cell scaffold material or a pericardial substitute" as used herein refers to reconstruction of the artificial material or the like into a tissue like an autogenous tissue, with a reduced immune reaction, after transplantation of an artificial material or the like into a living body.

EXAMPLES

Hereinbelow, the present invention will be described in more detail by way of examples, which however are in no way intended to limit the scope of the present invention.

EXAMPLE 1

Fibrosis inhibition effect by fibrosis inhibitor and fibrosis inhibition kit:
As decellularized tissues, a decellularized porcine small intestinal submucosa, a decellularized fetal rabbit skin, and a decellularized rabbit pericardium were used.

As a biocompatible polymer, fibrin was used. As for blood-derived fibrin, a fibrinogen solution was prepared by using a fibrinogen lyophilized powder and a fibrinogen dissolution in BOLHEAL for tissue adhesion (registered trade mark, KM Biologics Co., Ltd.). A thrombin solution was prepared by using a thrombin lyophilized powder and a thrombin dissolution in BOLHEAL for tissue adhesion (registered trade mark, KM Biologics Co., Ltd.). Recombinant fibrin was prepared using recombinant fibrinogen (Non-Patent Document 22) and recombinant thrombin (Non-Patent Document 23) both produced by a genetic engineering technique by KM Biologics Co., Ltd. As a dextrin gel, AdSpray (registered trade mark, Terumo Corporation) was used. The completion of the fixation of fibrin was confirmed when the transparent gel was slightly clouded, and the completion of the fixation of dextrin was confirmed when a white gel containing microbubbles was formed.

A cardiac fibrosis model was produced using a 5-to 6-month-old male Japanese white rabbit. An endotracheal tube was inserted into the trachea under anesthesia with xylazine and ketamine hydrochloride, and was then connected to an artificial respirator. A portion (5 cm) of a sternum was incised around notches for second to fifth costal cartilages. Pericardium (2.0 cm × 1.5 cm) immediately below the incised thorax part was incised. The epicardium was brought into contact with a metallic raspatory for 10 minutes. The sternum, muscle and skin in the incised thorax wound were sutured and closed. It was considered that the proper timing for the evaluation was a timing after an inflammation in the tissue ended and fibrosis was completed. Then, a long-term evaluation was performed in such a manner that the animal was raised in the conventional manner for 3 months and was then incised at the thorax, and the fibrosis grade of the epicardium was evaluated with naked eyes.

The fibrosis was rated in accordance with the following criteria:

Fibrosis grade 0: fibrosis was not observed.
Fibrosis grade 1: epicardium could be visually observed.
Fibrosis grade 2: epicardium could not be visually observed.

The fibrosis inhibition effect was determined by comparing a region with fibrosis grade 2 with a control group. The region (%) with fibrosis grade 2 was calculated in accordance with the formula:

$$(\text{the area of fibrosis grade 2})/(\text{the area of a pericardium}$$

$$\text{defect site}) \times 100.$$

In Example 1, the following test groups were compared and evaluated.

(1) A fibrosis inhibitor group (blood-derived fibrin)
(2) A fibrosis inhibition kit group (blood-derived fibrin and a decellularized porcine small intestinal submucosa)
(3) A fibrosis inhibition kit group (recombinant fibrin and a decellularized porcine small intestinal submucosa)
(4) A fibrosis inhibition kit group (recombinant fibrin and decellularized fetal rabbit skin)
(5) A fibrosis inhibition kit group (recombinant fibrin and decellularized rabbit pericardium)
(6) A fibrosis inhibition kit group (a dextrin gel and a decellularized porcine small intestinal submucosa)
(7) A control group (pericardium defect)
(8) A control group (autologous pericardium closure)
(9) A decellularized tissue alone group (a decellularized porcine small intestinal submucosa)
(10) A fibrin-decellularized tissue complex group (blood-derived fibrin was complexed with a decellularized porcine small-intestinal submucosa)

Groups (1) to (6) were groups of the invention, and groups (7) to (10) were control groups. With respect to the fibrin application method for (1) to (5), a fibrinogen solution and a thrombin solution were mixed together and the resultant mixture was applied to epicardium in a pericardium defect site using an application device, in which the final concentrations of the fibrinogen solution and the thrombin solution were 40 mg/mL and 125 U/mL, respectively, and the applied solutions were fixed. The dextrin gel preparation method and the dextrin gel application method for (6) were carried out as prescribed in a package insert for AdSpray. With respect to the decellularized tissue application method for (2) to (6) and (9), the decellularized tissue was sutured and fixed (prosthesis) to the remaining autologous pericardium in such a manner that the decellularized tissue was fixed to the pericardium defect site located between the epicardium and the sternum. With respect to the order of application for (2) to (6), fibrin or the dextrin gel was fixed to epicardium, and subsequently the decellularized tissue was sutured and fixed (prosthesis) to an autologous pericardium. Groups (7) to (10) were groups in each of which no fibrosis inhibitor was applied. Group (10) was a group in which fibrin was complexed with a decellularized tissue. The fibrinogen solution and the thrombin solution were mixed together and the resultant mixture was applied to both surfaces of the decellularized tissue to complex these components with each other, and the resultant product was sutured and fixed (prosthesis) to the remaining autologous pericardium in such a manner that the resultant product was fixed to a pericardium defect site located between the epicardium and the sternum. The results of the evaluation are shown in Table 1.

[Table 1]

| Test groups | Number of animals | The range (%) of fibrosis grade 2 in epicardium |
|---|---|---|
| (1) A fibrosis inhibitor group (blood-derived fibrin) | 4 | 0, 50, 10, 5 |
| (2) A fibrosis inhibition kit group (blood-derived fibrin and a decellularized porcine small intestinal submucosa) | 11 | 100, 20, 60, 30, 0, 10, 0, 0, 20, 20, 10 |
| (3) A fibrosis inhibition kit group (recombinant fibrin and a decellularized porcine small intestinal submucosa) | 6 | 0, 30, 30, 0, 0, 20 |
| (4) A fibrosis inhibition kit group (recombinant fibrin and decellularized fetal rabbit skin) | 6 | 30, 30, 0, 55, 0, 10 |
| (5) A fibrosis inhibition kit group (recombinant fibrin and decellularized rabbit pericardium) | 6 | 25, 50, 30, 20, 70, 20 |
| (6) A fibrosis inhibition kit group (a dextrin gel and a decellularized porcine small intestinal submucosa) | 6 | 10, 30, 40, 20, 100, 50 |
| (7) A control group (pericardium defect) | 11 | 100, 90, 70, 100, 70, 80, 0, 100, 100, 100, 5 |

(continued)

| Test groups | Number of animals | The range (%) of fibrosis grade 2 in epicardium |
| --- | --- | --- |
| (8) A control group (autologous pericardium closure) | 5 | 40, 70, 100, 80, 0 |
| (9) A decellularized tissue alone group (a decellularized porcine small intestinal submucosa) | 5 | 40, 90, 60, 0, 30 |
| (10) Fibrin-decellularized tissue complex group (blood-derived fibrin was complexed with decellularized porcine small intestinal submucosa) | 5 | 90, 100, 60, 100, 100 |

As apparent from the above-mentioned results, an excellent fibrosis inhibition effect was confirmed when the fibrosis inhibitors and the fibrosis inhibition kits which were products of the present invention were used. In each of the fibrosis inhibitors of the present invention, a significant difference ($p < 0.01$) was confirmed by Wilcoxon rank sum test when (3) the fibrosis inhibition kit group (recombinant fibrin and a decellularized porcine small intestinal submucosa) was compared with (7) control group (pericardium defect). When each of (1) the fibrosis inhibitor group (blood-derived fibrin), (2) the fibrosis inhibition kit group (blood-derived fibrin and a decellularized porcine small intestinal submucosa), (4) the fibrosis inhibition kit group (recombinant fibrin and a decellularized fetal rabbit skin) and (5) the fibrosis inhibition kit group (recombinant fibrin and a decellularized rabbit pericardium) was compared with (7) the control group (pericardium defect), a significant difference was confirmed ($p < 0.05$). When (3) the fibrosis inhibition kit group (recombinant fibrin and a decellularized porcine small intestinal submucosa) was compared with (8) the control group (autologous pericardium closure), a significant difference was confirmed ($p < 0.05$). When each of (2) the fibrosis inhibition kit group (blood-derived fibrin and a decellularized porcine small intestinal submucosa), (3) the fibrosis inhibition kit group (recombinant fibrin and a decellularized porcine small intestinal submucosa), (4) the fibrosis inhibition kit group (recombinant fibrin and a decellularized fetal rabbit skin) and (5) the fibrosis inhibition kit group (recombinant fibrin and a decellularized rabbit pericardium) was compared with (10) the fibrin-decellularized tissue complex group (blood-derived fibrin was complexed with a decellularized porcine small intestinal submucosa), a significant difference was confirmed ($p < 0.01$). When each of (1) the fibrosis inhibitor group (blood-derived fibrin) and (6) the fibrosis inhibition kit group (dextrin gel and a decellularized porcine small intestinal submucosa) was compared with (10) the fibrin-decellularized tissue complex group (blood-derived fibrin was complexed with a decellularized porcine small intestinal submucosa), a significant difference was confirmed ($p < 0.05$).

EXAMPLE 2

Adhesion inhibition effect by adhesion barrier kit:
As decellularized tissues, a decellularized porcine small intestinal submucosa, a decellularized fetal rabbit skin, and a rabbit decellularized pericardium were used.

As a biocompatible polymer, fibrin was used. As for blood-derived fibrin, a fibrinogen solution was prepared by using a fibrinogen lyophilized powder and a fibrinogen dissolution in BOLHEAL for tissue adhesion (registered trade mark, KM Biologies Co., Ltd.). A thrombin solution was prepared by using a thrombin lyophilized powder and a thrombin dissolution in BOLHEAL for tissue adhesion (registered trade mark, KM Biologics Co., Ltd.). Recombinant fibrin was prepared using recombinant fibrinogen (Non-Patent Document 22) and recombinant thrombin (Non-Patent Document 23) both produced by a genetic engineering technique by KM Biologics Co., Ltd. As a dextrin gel, AdSpray (registered trade mark, Terumo Corporation) was used. The completion of the fixation of fibrin was confirmed when the transparent gel was slightly clouded, and the completion of the fixation of dextrin was confirmed when a white gel containing microbubbles was formed.

A cardiac tissue adhesion model was produced using a 5- to 6-month-old male Japanese white rabbit. An endotracheal tube was inserted into the trachea under anesthesia with xylazine and ketamine hydrochloride, and was then connected to an artificial respirator. A portion (5 cm) of a sternum was incised around notches for second to fifth costal cartilages. Pericardium (2.0 cm × 1.5 cm) immediately below the incised thorax part was incised. The epicardium was brought into contact with a metallic raspatory for 10 minutes. The sternum, costa, muscle and skin in the incised thorax wound were sutured and closed. It was considered that the proper timing for the evaluation was a timing after an inflammation in the tissue ended and adhesion was completed. Then, a long-term evaluation was performed in such a manner that the animal was raised in the conventional manner for 3 months and was then incised at the thorax, and the adhesion at the epicardium abraded site was evaluated.

The adhesion was rated based in accordance with the criterial shown below depending on the level of detachment of the adhesion at an epicardium abraded site.

Adhesion grade 0: No adhesions
Adhesion grade 1: Mild adhesions (adhesions that do not require a blunt dissection to separate the space between the reconstruction ericardial and the sternal or the epicardium)
Adhesion grade 2: Moderate adhesions (adhesions requiring blunt dissection to separate the space between the reconstruction pericardial and the sternal or the epicardium)
Adhesion grade 3: Sever adhesions (adhesions requiring sharp dissection to separate the space between the reconstruction pericardial and the sternal or the epicardium)

The adhesion inhibition effect was determined by comparing a region in epicardium which had adhesion grade 0 and a region in the epicardium which had adhesion grade 3 with control groups. A region (%) in the epicardium which had adhesion grade 0 was calculated in accordance with the formula:

$$\text{(the area in epicardium which had adhesion grade 0)/(the area of a pericardium defect site)} \times 100.$$

A region (%) in the epicardium which had adhesion grade 3 was calculated in accordance with the formula: (the area in epicardium which had adhesion grade 3)/(the area of a pericardium defect site) $\times$ 100.
In Example 2, the following test groups were compared and evaluated.

(1) An adhesion barrier kit group (blood-derived fibrin and a decellularized porcine small intestinal submucosa)
(2) An adhesion barrier kit group (recombinant fibrin and a decellularized porcine small intestinal submucosa)
(3) Adhesion barrier kit group (recombinant fibrin and decellularized fetal rabbit skin)
(4) An adhesion barrier kit group (recombinant fibrin and a decellularized rabbit pericardium)
(5) An adhesion barrier kit group (dextrin gel and a decellularized porcine small intestinal submucosa)
(6) A control group (pericardium defect)
(7) A control group (autologous pericardium closure)
(8) A fibrin-decellularized tissue complex group (blood-derived fibrin was complexed with a decellularized porcine small intestinal submucosa)

Groups (1) to (5) were groups of the invention, and groups (6) to (8) were control groups. With respect to the fibrin application method for (1) to (4), a fibrinogen solution and a thrombin solution were mixed together and the resultant mixture was applied and fixed to epicardium in a pericardium defect site using an application device, in which the final concentrations of the fibrinogen solution and the thrombin solution were 40 mg/mL and 125 U/mL, respectively. The dextrin gel preparation method and the application method for (5) were carried out as prescribed in a package insert for AdSpray. With respect to the decellularized tissue application method for (1) to (5), the decellularized tissue was sutured and fixed (prosthesis) to the remaining autologous pericardium so as to be fixed to the pericardium defect site located between the epicardium and the sternum. With respect to the order of the applications for (1) to (5), fibrin or a dextrin gel was fixed to epicardium, and subsequently a decellularized tissue was sutured and fixed to an autologous pericardium. Groups (6) to (8) were groups in each of which no adhesion barrier kit was applied. Group (8) was a group in which fibrin was complexed with a decellularized tissue. The fibrinogen solution and the thrombin solution were mixed together and the resultant mixture was applied to both surfaces of the decellularized tissue to complex these components with each other, and the resultant product was sutured and fixed (prosthesis) to the remaining autologous pericardium in such a manner that the resultant product was fixed to a pericardium defect site located between the epicardium and the sternum. The results of the evaluation are shown in Table 2.

[Table 2]

| Test group | Number of animals | Region (%) of adhesion grade 0 in epicardium | Region (%) of adhesion grade 3 in epicardium |
|---|---|---|---|
| (1) Adhesion barrier kit group (blood-derived fibrin and decellularized porcine small intestinal submucosa) | 11 | 100, 100, 70, 50, 100, 0, 30, 100, 30, 0, 70 | 0, 0, 0, 0, 0, 0, 0, 0, 5, 20, 0 |

(continued)

| Test group | Number of animals | Region (%) of adhesion grade 0 in epicardium | Region (%) of adhesion grade 3 in epicardium |
|---|---|---|---|
| (2) Adhesion barrier kit group (recombinant fibrin and decellularized porcine small intestinal submucosa) | 6 | 100, 0, 40, 100, 90, 100 | 0, 0, 0, 0, 0, 0 |
| (3) Adhesion barrier kit group (recombinant fibrin and decellularized fetal rabbit skin) | 6 | 90, 20, 100, 100, 100, 60 | 0, 5, 0, 0, 0, 20 |
| (4) Adhesion barrier kit group (recombinant fibrin and decellularized rabbit pericardium) | 6 | 100, 0, 0, 100, 40, 95 | 0, 20, 20, 0, 0, 0 |
| (5) Adhesion barrier kit group (dextrin gel and decellularized porcine small intestinal submucosa) | 6 | 0, 50, 100, 30, 0, 95 | 0, 0, 0, 20, 20, 0 |
| (6) Control group (pericardium defect) | 11 | 0, 0, 0, 0, 30, 0, 95, 20, 0, 70, 50 | 30, 100, 50, 80, 70, 20, 0, 80, 60, 30, 10 |
| (7) Control group (autologous pericardium closure) | 5 | 0, 0, 0, 0, 100 | 0, 10, 80, 100, 0 |
| (8) Fibrin-decellularized tissue complex group (blood-derived fibrin is complexed with decellularized porcine small intestinal submucosa) | 5 | 0, 0, 0, 5, 0 | 40, 0, 0, 0, 0 |

As apparent from the above-mentioned results, when the adhesion barrier kits which were products of the present invention were used, a long-term excellent adhesion inhibition effect was confirmed. The regions (%) of the adhesion grade 0 in epicardium were compared. As a result, a significant difference ($p < 0.01$) was confirmed in the adhesion barrier kit of the present invention by Wilcoxon rank sum test when comparison was made between (3) the adhesion barrier kit group (recombinant fibrin and decellularized fetal rabbit skin) with (6) the control group (pericardium defect). When each of (1) the adhesion barrier kit group (blood-derived fibrin and a decellularized porcine small intestinal submucosa) and (2) the adhesion barrier kit group (recombinant fibrin and a decellularized porcine small intestinal submucosa) was compared with (6) the control group (pericardium defect), a significant difference was confirmed ($p < 0.05$). When (3) the adhesion barrier kit group (recombinant fibrin and decellularized fetal rabbit skin) was compared with (7) the control group (autologous pericardium closure), a significant difference was confirmed ($p < 0.05$). When each of (1) the adhesion barrier kit group (blood-derived fibrin and a decellularized porcine small intestinal submucosa) and (2) the adhesion barrier kit group (recombinant fibrin and a decellularized porcine small intestinal submucosa) was compared with (8) the fibrin-decellularized tissue complex group (blood-derived fibrin was complexed with a decellularized porcine small intestinal submucosa), a significant difference was confirmed ($p < 0.05$) . The regions (%) of the adhesion grade 3 in epicardium were compared. As a result, a significant difference ($p < 0.01$) was confirmed in the adhesion barrier kits of the present invention by Wilcoxon rank sum test when each of (1) the adhesion barrier kit group (blood-derived fibrin and a decellularized porcine small intestinal submucosa), (2) the adhesion barrier kit group (recombinant fibrin and a decellularized porcine small intestinal submucosa), (3) the adhesion barrier kit group (recombinant fibrin and a decellularized fetal rabbit skin), (4) the adhesion barrier kit group (recombinant fibrin and a decellularized rabbit epicardium) and (5) the adhesion barrier kit group (a dextrin gel and a decellularized porcine small intestinal submucosa) was compared with (6) the control group (pericardium defect). When (2) the adhesion barrier kit group (recombinant fibrin and a decellularized porcine small intestinal submucosa) was compared with (7) the control group (autologous pericardium closure), a significant difference was confirmed ($p < 0.05$).

EXAMPLE 3

Promotion of self-organization of decellularized tissue by fibrosis inhibition kit and adhesion barrier kit:
As the decellularized tissue, a decellularized porcine small intestinal submucosa was used.
As a biocompatible polymer, fibrin was used. As for blood-derived fibrin, a fibrinogen solution was prepared by using a fibrinogen lyophilized powder and a fibrinogen dissolution in BOLHEAL for tissue adhesion (registered trade mark, KM Biologics Co., Ltd.). A thrombin solution was prepared by using a thrombin lyophilized powder and a thrombin dissolution in BOLHEAL for tissue adhesion (registered trade mark, KM Biologics Co., Ltd.). Recombinant fibrin was prepared using recombinant fibrinogen (Non-Patent Document 22) and recombinant thrombin (Non-Patent Document 23) both produced by a genetic engineering technique by KM Biologics Co., Ltd. The completion of the fixation of fibrin was confirmed when the transparent gel was slightly clouded, and the completion of the fixation of dextrin was confirmed when a white gel containing microbubbles was formed.

The self-organization of a decellularized tissue was evaluated using a rabbit cardiac model. A 5- to 6-month-old male Japanese white rabbit was used. An endotracheal tube was inserted into the trachea under anesthesia with xylazine and ketamine hydrochloride, and was then connected to an artificial respirator. A portion (5 cm) of a sternum was incised around notches for second to fifth costal cartilages. Pericardium (2.0 cm × 1.5 cm) immediately below the incised thorax part was incised. The epicardium was brought into contact with a metallic raspatory for 10 minutes. The sternum, costa, muscle and skin in the incised thorax wound were sutured and closed. For a long-term evaluation, the rabbit was subjected to the conventional raising for 3 months and was then subjected to an incision that opened the thorax to collect a decellularized tissue. For evaluating the strength of the decellularized tissue, the breakage of the decellularized tissue upon the collection was evaluated. The breakage occurrence rate (%) was calculated in accordance with the following formula:

```
(the number of individuals in which the decellularized tissue
was broken upon the collection)/(the number of individuals
in each test group) × 100.
```

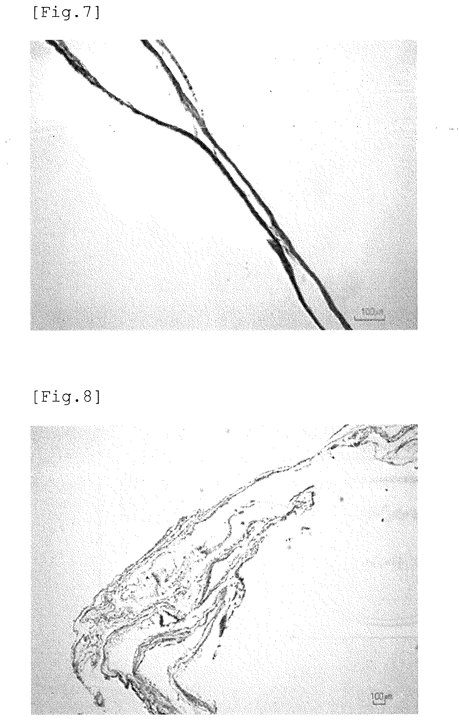

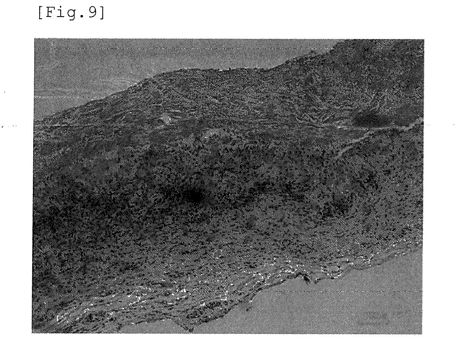

A histopathological specimen of the collected decellularized tissue was produced, and was then subjected to HE staining. In this manner, the degree of self-organization was evaluated. The pathological photographs of the results are shown in Figs. 7 to 9.

In Example 3, the following test groups were evaluated.

(1) A fibrosis inhibition and adhesion barrier kit group (blood-derived fibrin and a decellularized porcine small intestinal submucosa)
(2) A decellularized tissue alone group (a decellularized porcine small intestinal submucosa)
(3) A fibrin-decellularized tissue complex group (blood-derived fibrin was complexed with a decellularized porcine small intestinal submucosa).

With respect to the method for applying the decellularized tissue in (1) and (2), the decellularized tissue was sutured and fixed (prosthesis) to the remaining autologous pericardium so that the decellularized tissue was fixed to the pericardium defect site located between epicardium and a sternum. Group (3) was a group in which fibrin was complexed with a decellularized tissue The complexed product was sutured and fixed (prosthesis) to the remaining autologous pericardium in such a manner that the complexed product was fixed to a pericardium defect site located between epicardium and a sternum. The results of the evaluation are shown in Table 3.

[Table 3]

| Test group | Number of animals | Occurrence rate of breakage of decellularize d tissue (%) | Self-organization (histopathologic al evaluation) |
|---|---|---|---|
| (1) A fibrosis inhibition and adhesion barrier kit group (blood-derived fibrin and a decellularized porcine small intestinal submucosa) | 11 | 0 | In many individuals, inflammatory cells were reduced and self-organization proceeded. |
| (2) A decellularized tissue alone group (a decellularized porcine small intestinal submucosa) | 5 | 40 | In many individuals, inflammatory cells were reduced, but tissues were sparse and it was considered that the strength of the tissues was brittle. |
| (3) A fibrin-decellularized tissue complex group (blood-derived fibrin was complexed with a decellularized porcine small intestinal submucosa) | 5 | 20 | In many individuals, many inflammatory cells were still found and self-organization was not induced yet. |

As apparent from the above-mentioned results, it was confirmed that, the fibrosis inhibition and adhesion barrier kit which was a product of the present invention can promote the self-organization of a decellularized tissue to reconstruct an autologous pericardium-like tissue having proper strength.

INDUSTRIAL APPLICABILITY

The present invention can be used as a fibrosis inhibitor for the surface of an organ or a tissue, and can also be used for the inhibition of adhesion of tissues.

**Claims**

1.  tissue fibrosis inhibitor comprising a biocompatible polymer.

2.  The fibrosis inhibitor according to claim 1, wherein the biocompatible polymer is fibrin or a dextrin gel.

3.  A tissue fibrosis inhibition kit comprising a combination of a biocompatible polymer and a cell scaffold material.

4.  The fibrosis inhibition kit according to claim 3, wherein the biocompatible polymer is fixed to one tissue fibrosis of which is to be inhibited, and prosthesis is made with the cell scaffold material for a tissue defect site in the other tissue.

5.  The fibrosis inhibition kit according to claim 3 or 4, wherein the biocompatible polymer is fibrin or a dextrin gel.

6.  The fibrosis inhibition kit according to any one of claims 3 to 5, wherein the cell scaffold material is a decellularized tissue.

7.  The fibrosis inhibition kit according to claim 6, wherein the decellularized tissue is a tissue produced by decellularizing a biological tissue selected from the group consisting of a small intestinal submucosa, pericardium, bladder, amnion, dura mater, peritoneum, greater omentum, thoracic diaphragm, fascia, dermis and skin.

8.  The fibrosis inhibition kit according to any one of claims 3 to 7, which promotes the self-organization of the cell scaffold material.

9.  The fibrosis inhibition kit according to any one of claims 3 to 8, wherein the tissue fibrosis of which is to be inhibited is epicardium.

10.  A tissue fibrosis inhibition kit comprising a combination of a biocompatible polymer and a pericardial substitute.

11.  The fibrosis inhibition kit according to claim 10, wherein the biocompatible polymer is fixed to one tissue fibrosis of which is to be inhibited, and prosthesis is made with the pericardial substitute for a tissue defect site in the other tissue.

12.  The fibrosis inhibition kit according to claim 10 or 11, wherein the biocompatible polymer is fibrin or a dextrin gel.

13.  The fibrosis inhibition kit according to any one of claims 10 to 12, wherein the pericardial substitute is a decellularized tissue.

14.  The fibrosis inhibition kit according to claim 13, wherein the decellularized tissue is a tissue produced by decellularizing a biological tissue selected from the group consisting of a small intestinal submucosa, pericardium, bladder, amnion, dura mater, peritoneum, greater omentum, thoracic diaphragm, fascia, dermis and skin.

15.  The fibrosis inhibition kit according to any one of claims 10 to 14, which promotes the self-organization of the pericardial substitute.

16.  The fibrosis inhibition kit according to any one of claims 10 to 15, wherein the tissue fibrosis of which is to be inhibited is pericardium.

17.  An adhesion barrier kit, wherein the biocompatible polymer is fixed to one tissue fibrosis of which is to be inhibited, and prosthesis is made with the cell scaffold material or the pericardial substitute for a tissue defect site in the other tissue.

18. The adhesion barrier kit according to claim 17, wherein the biocompatible polymer is fibrin or a dextrin gel.

19. The adhesion barrier kit according to claim 17, wherein the cell scaffold material or the pericardial substitute is a decellularized tissue.

20. The adhesion barrier kit according to claim 19, wherein the decellularized tissue is a tissue produced by decellularizing a biological tissue selected from the group consisting of a small intestinal submucosa, pericardium, bladder, amnion, dura mater, peritoneum, greater omentum, thoracic diaphragm, fascia, dermis and skin.

21. The adhesion barrier kit according to any one of claims 17 to 20, which promotes the self-organization of the cell scaffold material or the pericardial substitute.

22. The adhesion barrier kit according to any one of claims 17 to 21, wherein the tissue to be inhibited from adhesion is epicardium.

EP 4 008 368 A1

[Fig. 1]

[Fig.2]

15

[Fig.3]

[Fig.4]

[Fig.5]

[Fig.6]

[Fig.7]

[Fig.8]

[Fig.9]

## INTERNATIONAL SEARCH REPORT

| International application No. |
| --- |
| PCT/JP2020/029532 |

**A. CLASSIFICATION OF SUBJECT MATTER**
A61L 27/22(2006.01)i; A61K 35/12(2015.01)i; A61K 35/22(2015.01)i; A61K 35/30(2015.01)i; A61K 35/34(2015.01)i; A61K 35/36(2015.01)i; A61K 35/38(2015.01)i; A61K 35/50(2015.01)i; A61K 38/36(2006.01)i; A61L 27/36(2006.01)i; A61L 27/58(2006.01)i; A61P 9/00(2006.01)i; A61P 43/00(2006.01)i
FI:  A61L27/22; A61P9/00; A61P43/00 105; A61K35/38; A61K35/22; A61K35/50; A61K35/30; A61K35/12; A61K35/34; A61K35/36; A61K38/36; A61L27/58; A61L27/36 400

According to International Patent Classification (IPC) or to both national classification and IPC

**B. FIELDS SEARCHED**

Minimum documentation searched (classification system followed by classification symbols)
A61L27/22; A61K35/12; A61K35/22; A61K35/30; A61K35/34; A61K35/36; A61K35/38; A61K35/50; A61K38/36; A61L27/36; A61L27/58; A61P9/00; A61P43/00

Documentation searched other than minimum documentation to the extent that such documents are included in the fields searched
Published examined utility model applications of Japan 1922–1996
Published unexamined utility model applications of Japan 1971–2020
Registered utility model specifications of Japan 1996–2020
Published registered utility model applications of Japan 1994–2020

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | MORO, H. et al., "The effect of fibrin glue on inhibition of pericardial adhesions", The Japanese Journal of Thoracic and Cardiovascular Surgery, 1999, 47, pp. 79-84, in particular, abstract, page 82, left column, paragraph [0003] to page 83, left column, paragraph [0001], fig. 3 | 1-2<br>3-22 |
| X<br>A | JP 6-508356 A (GLIATECH, INC.) 22 September 1994 (1994-09-22) in particular, claims, examples | 1-2<br>3-22 |
| X<br>A | JP 2003-528026 A (ML LABORATORIES PLC) 24 September 2003 (2003-09-24) in particular, claims | 1-2<br>3-22 |

☒ Further documents are listed in the continuation of Box C.    ☒ See patent family annex.

| | |
| --- | --- |
| *  Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 28 September 2020 (28.09.2020) | 27 October 2020 (27.10.2020) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

## INTERNATIONAL SEARCH REPORT

International application No.

PCT/JP2020/029532

C (Continuation).   DOCUMENTS CONSIDERED TO BE RELEVANT

| Category* | Citation of document, with indication, where appropriate, of the relevant passages | Relevant to claim No. |
|---|---|---|
| X<br>A | FUNAMOTO, S. et al., "A fibrin-coated pericardial extracellular matrix prevented heart adhesion in a rat model", J Biomed Mater Res Part B., 19 September 2018, 2019, 107B, pp. 1088-1094, in particular, abstract | 3, 5-10, 12-16<br>4, 11, 17-22 |
| X<br>A | WO 2016/143746 A1 (JURIDICAL FOUNDATION THE CHEMO-SERO-THERAPEUTIC RESEARCH INSTITUTE) 15 September 2016 (2016-09-15) in particular, claims, examples | 3, 5-10, 12-16<br>4, 11, 17-22 |

Form PCT/ISA/210 (continuation of second sheet) (January 2015)

**INTERNATIONAL SEARCH REPORT**

Information on patent family members

International application No.

PCT/JP2020/029532

| Patent Documents referred in the Report | Publication Date | Patent Family | Publication Date |
|---|---|---|---|
| JP 6-508356 A | 22 Sep. 1994 | US 5605938 A in particular, claims, examples US 5705177 A US 5705178 A US 5994325 A US 6020326 A US 6083930 A US 6127348 A US 6417173 B1 US 2003/0069205 A1 WO 1992/021354 A1 EP 586535 A1 EP 1027893 A2 EP 1038528 A1 JP 2000-312715 A JP 2002-138039 A JP 2002-138040 A JP 2002-154972 A JP 2002-154973 A | |
| JP 2003-528026 A | 24 Sep. 2003 | US 2007/0167404 A1 in particular, claims US 7732428 B1 US 2010/0240607 A1 US 2012/0115810 A1 WO 1999/058168 A1 EP 1085920 A1 JP 2005-47929 A US 2018/0078679 A1 | |
| WO 2016/143746 A1 | 15 Sep. 2016 | in particular, claims, examples EP 3269402 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11502431 T
- JP 2001327592 A
- JP 2003500170 T
- JP 4092397 B
- JP 2008111530 A
- JP 2009050297 A

**Non-patent literature cited in the description**

- **TSUNEO FUKUSHIMA et al.** *Surgical Therapy,* 2006, vol. 94 (6), 919-924
- **UYAMA N. et al.** *Sci Rep.,* 2019, vol. 9 (1), 17558
- **DOBELL AR. et al.** *Ann Thorac Surg.,* 1984, vol. 37, 273-8
- **OZEREN M. et al.** *Cardiovasc. Surg.,* 2002, vol. 10 (5), 489-493
- **TETSUYA KITAGAWA.** *PEDIATRIC CARDIOLOGY and CARDIAC SURGERY,* 2004, vol. 20 (6), 632-633
- **LIERMANN A. et al.** *Helv Chir Acta.,* 1992, vol. 58, 515-519
- **MURALIDHARAN S. et al.** *J Biomed Mater Res.,* 1991, vol. 25, 1201-1209
- **BUNTON RW. et al.** *J Thorac Cardiovasc Surg.,* 1990, vol. 100, 99-107
- **JUKKA T. et al.** *Interactive cardiovascular and thoracic surgery.,* 2011, vol. 12 (2), 270-272
- **KAUSHAL S. et al.** *Thorac Cardiovasc Surg.,* 2011, vol. 141, 789-795
- **NAITO Y. et al.** *J Thorac Cardiovasc Surg.,* 2008, vol. 135, 850-856
- **TAKAMICHI SATO et al.** *Obstetrics and Gynecology,* 1990, vol. 57 (12), 2398-2404
- **KIYOSHI OKUDA et al.** *The World of Obstetrics and Gynecology,* 1993, vol. 45 (9), 759-764
- **AKIO KISHIDA.** *Organ Biology,* 2018, vol. 25 (1), 27-34
- **SINGELYN J.M. et al.** *Biomaterials,* 2009, vol. 30, 5409-5416
- **SINGELYN J.M et al.** *J. Am. Coll. Cardiol.,* 2012, vol. 59, 751-763
- **SONYA B. et al.** *Sci. Transl. Med.,* 2013, vol. 5, 173ra25
- **SASAKI S. et al.** *Mol. Vis.,* 2009, vol. 15, 2022-2028
- **YOSHIHIDE H. et al.** *Biomaterials,* 2010, vol. 31, 3941-3949
- **SEIICHI F. et al.** *Biomaterials,* 2010, vol. 31, 3590-3595
- **NEGISHI J. et al.** *J. Artif. Organs,* 2011, vol. 14, 223-231
- **HIRASHIMA M. et al.** *J. Biochem.,* 2016, vol. 159, 261-270
- **META A. et al.** *J. Biosci. Bioeng,* 2015, vol. 120, 432-437